# EUROPÄISCHE PATENTSCHRIFT

(11) **EP 2 666 492 B1**
(45) Veröffentlichungstag und Bekanntmachung des Hinweises auf die Patenterteilung: **14.10.2015**
(21) Anmeldenummer: 13167902.9
(22) Anmeldetag: 15.05.2013
(51) Int. Cl.: A61B 5/00, A61M 1/36, A61B 5/1455

(54) **Medizinisches Gerät zur extrakorporalen Blutbehandlung mit mehreren Sensoreinheiten**
Medical device for extracorporeal blood treatment with multiple sensor units
Appareil médical de traitement extracorporel du sang doté de plusieurs unités de capteurs

(30) Priorität: 23.05.2012 DE 102012104461
(43) Veröffentlichungstag der Anmeldung: 27.11.2013
(73) Patentinhaber: B. Braun Avitum AG, 34212 Melsungen (DE)
(72) Erfinder: Schade, Andreas, 36199 Rotenburg (DE)
(74) Vertreter: Winter, Brandl, Fürniss, Hübner, Röss, Kaiser, Polte - Partnerschaft mbB

(56) Entgegenhaltungen:
- EP-A2- 0 720 013
- WO-A1-2010/136962
- US-A- 5 685 989
- US-A- 6 144 444
- US-A1- 2011 009 800

## Beschreibung

Die Erfindung betrifft ein medizinisches Gerät zur extrakorporalen Blutbehandlung, umfassend einen extrakorporalen Blutkreislauf mit wenigstens einem Schlauch und wenigstens zwei Sensoreinheiten mit unterschiedlichen Wirkprinzipien, an denen Abschnitte des Schlauchs für eine Messung während der Therapie ankoppelbar sind.

Medizinische Geräte mit Merkmalen des Anspruchs 1 sind beispielsweise aus WO 2010/136962 A1 und US 6,144,444 A bekannt. In beiden Druckschriften werden Vorrichtungen beschrieben, die durch einen Schlauch fließendes Blut elektromagnetischen Wellen aussetzen und anhand der reflektierten beziehungsweise transmittierten Wellen bestimmte Werte des Blutes ermitteln. Die in WO 2010/136962 A1 und US 6,144,444 A offenbarten Vorrichtungen sind Multisensor-Einheiten mit jeweils einem einzigen sensorneutralen Bauteil.

Bei medizintechnischen Geräten zur extrakorporalen Blutbehandlung wird über Schläuche und Kammern ein extrakorporaler Blutkreislauf aufgebaut, wobei hierzu in der Regel ein Einmalartikel in Form eines Schlauchs verwendet wird, welcher vom Bediener für eine Therapie am Gerät befestigt wird. Damit der Bediener die Befestigung in der vorgesehenen Art und Weise vornehmen kann, sind entsprechende Haltevorrichtungen am Gerät angebracht. Ferner werden bei der Behandlung von Patienten verschiedene Parameter mit Hilfe von Sensoren bzw. Messsystemen erfasst. Da in der Regel ein direkter Kontakt der Sensoren mit dem Blut des Patienten nicht gewünscht bzw. nicht notwendig ist, werden die Messungen häufig an einem Schlauchabschnitt des Einmalartikels durchgeführt. Um die gewünschten Messergebnisse zu erzielen, kann es dabei notwendig sein, den verwendeten Einmalartikel in einer gewünschten Position am Gerät und dem Sensor anzubringen.

Da der Einmalartikel vom Bediener des Geräts am Gerät angebracht wird, obliegt es ihm auch, diesen in der vorgesehenen und technisch notwendigen Art und Weise zu verlegen. Hierbei kann jedoch vom Bediener nicht unbedingt erwartet werden, dass er die Funktionsweise des jeweiligen Messsystems kennt und entsprechend berücksichtigen kann. Daher empfiehlt es sich, die vom Bediener auszuführenden Handlungschritte zum Einlegen des Einmalartikels für auf dem Schlauch messende Systeme zu vereinheitlichen.

An den bekannten Geräten sieht sich der Bediener jedoch häufig an verschiedenen Messsystemen mit unterschiedlichen und für ihn nicht direkt nachvollziehbaren Handlungsschritten konfrontiert. Zudem können Abweichungen von der vorgesehenen Handlungsweise negative Auswirkungen auf die Funktionsweise der Messsysteme haben.

Aufgabe der Erfindung ist es daher, ein medizinisches Gerät zur extrakorporalen Blutbehandlung bereitzustellen, das wenigstens zwei auf einem Schlauch messende Sensoreinheiten mit unterschiedlichen Wirkprinzipien aufweist, an welche ein Schlauchabschnitt des extrakorporalen Blutkreislaufs für eine Messung während der Therapie anzukoppeln ist, wobei die für die Anbringung des Schlauchs erforderlichen Handlungsschritte vereinheitlicht sein sollen.

Erfindungsgemäß wird diese Aufgabe durch ein medizinisches Gerät zur extrakorporalen Blutbehandlung gemäß dem unabhängigen Anspruch 1 gelöst. Vorteilhafte Weiterbildungen des Geräts ergeben sich aus den Unteransprüchen 2-10.

Das erfindungsgemäße medizinische Gerät zur extrakorporalen Blutbehandlung umfasst einen extrakorporalen Blutkreislauf mit wenigstens einem Schlauch und wenigstens zwei auf dem Schlauch messende Sensoreinheiten mit unterschiedlichen Wirkprinzipien, an denen Abschnitte des Schlauchs für eine Messung ankoppelbar sind. Jede Sensoreinheit besteht dabei aus einem sensorspezifischen Bauteil und einem sensorneutralen Bauteil, wobei die sensorneutralen Bauteile der Sensoreinheiten identisch ausgebildet sind, während sich die sensorspezifischen Bauteile abhängig vom jeweiligen Wirkprinzip unterscheiden und jeweils eine spezifische Sensorik umfassen. Ferner ist für jede Sensoreinheit ein sensorneutrales Bauteil auf einem sensorspezifischen Bauteil montiert, wobei ein Abschnitt des Schlauchs bei Einbringung und Fixierung in einem sensorneutralen Bauteil so an die Sensorik des darunter liegenden sensorspezifischen Bauteils ankoppelbar ist, dass mit der Sensorik eine Messung am Schlauch durchführbar ist.

Die sensorneutralen Bauteile jeder Sensoreinheit sind somit unabhängig vom Wirkprinzip der zugehörigen Sensorik identisch und nur die sensorspezifischen Bauteile unterscheiden sich voneinander. Von außen stellt sich dem Bediener dann jede Sensoreinheit gleich dar, da die sensorneutralen Bauteile über den sensorspezifischen Bauteilen montiert sind. Die sensorneutralen Bauteile geben dabei die Vorgehensweise für ein Einlegen und Fixieren eines Schlauchabschnitts innerhalb der Sensoreinheit vor und diese ist für jede Sensoreinheit gleich. Der Aufbau der Sensoreinheit gewährleistet dabei jedoch, dass der Schlauchabschnitt gleichzeitig so an eine unterhalb des sensorneutralen Bauteils angeordnete Sensorik angekoppelt wird, dass eine Messung durchführbar ist. Der Bediener muss somit beim Einlegen eines Schlauchs nicht wissen, wie die Funktionsweise der jeweiligen Sensorik ist, sondern er kann den Schlauch in jede Sensoreinheit auf dieselbe Art und Weise eingelegen, und der Aufbau der Sensoreinheit führt automatisch zu einer Ankopplung des Schlauchs an die jeweilige Sensorik.

In einem Ausführungsbeispiel der Erfindung weisen die sensorneutralen und sensorspezifischen Bauteile jeweils unterschiedlich ausgeformte Grundkörper auf, und die Grundkörper der sensorneutralen Bauteile umschließen jeweils die darunter liegenden Grundkörper der sensorspezifischen Bauteile. So ist bei der Montage einer solchen Sensoreinheit auf dem Gehäuse eines medizinischen Geräts von außen nur das sensorneutrale Bauteil zu sehen.

Vorzugsweise weisen die Grundkörper der sensorneutralen Bauteile dabei eine Aussparung auf, und ein Abschnitt des Schlauchs ist so in die sensorneutralen Bauteile einbringbar, dass der Abschnitt des Schlauchs durch die Aussparung hindurch an die Sensorik eines sensorspezifischen Bauteils ankoppelbar ist. So wird bei übereinander montierten Bauteilen gewährleistet, dass ein in das obere Bauteil eingelegter Schlauchabschnitt für eine Messung an die darunter liegende Sensorik ankoppelbar ist.

In einem Ausführungsbeispiel der Erfindung weist jedes sensorspezifische Bauteil eine Schlauchrinne auf, in welche der Abschnitt eines Schlauchs einlegbar ist. So kann der Schlauch gegenüber der Sensorik ausgerichtet und fixiert werden. Dabei können die Schlauchrinnen von wenigstens zwei sensorspezifischen Bauteilen auch unterschiedlich ausgebildet sein. Beispielsweise können sie verschiedene Bohrungen zur Ankopplung von Sensoren an den Schlauch oder sonstige Mittel aufweisen, welche für eine Messung erforderlich sind.

Um die Schlauchrinne eines sensorspezifischen Bauteils so mit dem zugehörigen sensorneutralen Bauteil zu verbinden, dass ein in das sensorneutrale Bauteil eingelegter Schlauch vorschriftsmäßig innerhalb der Schlauchrinne zum Liegen kommt und an die Sensorik angekoppelt ist, kann jedes sensorneutrale Bauteil zwei rinnenförmige Schlauchhalterungen mit der dazwischen liegenden Aussparung aufweisen. Diese Schlauchhalterungen liegen dann vorzugsweise so an den beiden Enden der Schlauchrinne des sensorspezifischen Bauteils an, dass sich eine durchgehende Schlauchrinne ergibt. Für den Bediener stellt sich die Kombination aus zwei Schlauchhalterungen und der dazwischen liegenden Schlauchrinne des sensorspezifischen Bauteils somit als durchgehende Schlauchrinne dar, in welche der Schlauch einzulegen ist.

Um einen eingelegten Schlauch innerhalb der Sensoreinheit zu fixieren, kann jedes sensorneutrale Bauteil eine Abdeckung aufweisen, mit welcher der Abschnitt eines Schlauchs in einer geschlossenen und arretierten Stellung gegen die Sensorik des zugehörigen sensorspezifischen Bauteils drückbar ist. Wird eine solche Abdeckung angebracht, liegt sie somit am Schlauch an und bewirkt eine Druckkraft auf den Schlauch in Richtung der Sensorik. So kann der Schlauch innerhalb einer Schlauchrinne fixiert werden. Um diese Druckkraft gezielt aufbringen zu können, kann die Abdeckung wenigstens ein an der Abdeckung hervorstehendes Druckelement aufweisen, über welches Druck auf den Abschnitt eines Schlauchs aufbringbar ist.

Der Schlauch kann jedoch auch auf andere Arten an der Sensoreinheit fixiert werden. Hierbei kommen beispielsweise Bügel, Klemmelemente, elastische Spannmittel, etc. in Betracht. Auch der Druck auf den Schlauch innerhalb einer Schlauchrinne kann neben einem Druckelement mit ergänzenden oder alternativen Mitteln erzeugt werden.

Ist dagegen die beschriebene Abdeckung vorgesehen, ist diese vorzugsweise schwenkbar an dem jeweiligen Grundkörper des sensorneutralen Bauteils angebracht und in der geschlossenen Stellung an dem Grundkörper arretierbar. Für diese Arretierung kann an der Abdeckung und dem Grundkörper jeweils wenigstens ein Rastelement vorgesehen ist. Wird die Abdeckung in der geschlossenen Stellung gegen den Grundkörper des sensorneutralen Bauteils gedrückt, rasten diese Rastelemente ein und halten die Abdeckung in dieser Stellung. Sie sind jedoch so ausgebildet, dass sich die Verrastung durch ein Ziehen an der Abdeckung und/oder ein Drücken auf die Rastelemente manuell wieder lösen lässt.

Dabei ist die Verschlussmechanik der Sensoreinheit vorzugsweise ausschließlich an dem sensorneutralen Bauteil ausgebildet. Dies gilt auch für gegebenenfalls notwendige Abdichtungen der Sensoreinheit nach außen.

Die Erfindung ermöglicht es so, auf dem Schlauch messende Systeme mit verschiedenen Wirkprinzipien für den Bediener einheitlich zu gestalten. Insbesondere ist dies für die Einbindung von optischen und/oder thermischen Sensoriken, Ultraschallsensoren, taktilen Sensoren und/oder Drucksensoren vorteilhaft. Bedienungsbedingte Messfehler werden hierdurch ausgeschlossen oder zumindest stark reduziert, und der Bediener muss sich nur einen Handlungsablauf einprägen, da die Sensoreinheiten des medizinischen Geräts zur extrakorporalen Blutbehandlung ein einheitliches Erscheinungsbild und eine klare Semantik haben. Dabei sind die vom Bediener auszuführenden Handlungsschritte unabhängig vom Messprinzip der jeweiligen Sensoreinheit.

Ferner führt die Verwendung von Gleichteilen zu einer Kostenreduzierung, denn die identischen sensorneutralen Bauteile können kostengünstig hergestellt und für mehrere Sensoreinheiten verwendet werden. Dies ist insbesondere gegenüber einer Lösung vorteilhaft, bei der mehrere Sensoreinheiten eines medizinisches Geräts zwar jeweils die gleiche Schlaucheinlegung ermöglichen, jedoch hierfür jede Sensoreinheit eine spezielle Bauteilkonfiguration aufweisen muss, um gleichzeitig eine Ankopplung des Schlauchs an die jeweilige Sensorik zu ermöglichen. Eine solche Lösung würde eine geringere Verwendung von Gleichteilen ermöglichen.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: eine schematische Seitenansicht eines Ausführungsbeispiels eines sensorspezifischen Bauteils;
- Fig. 2: eine schematische Seitenansicht eines Ausführungsbeispiels eines sensorneutralen Bauteils;
- Fig. 3: eine schematische Aufsicht auf ein sensorneutrales Bauteil mit geöffneter Abdeckung;
- Fig. 4: eine schematische Seitenansicht einer montierter Sensoreinheit mit eingebrachtem Schlauch;
- Fig. 5: eine Sensoreinheit beim Einlegen eines Schlauchs; und
- Fig. 6: eine Sensoreinheit beim Schließen der Abdeckung.

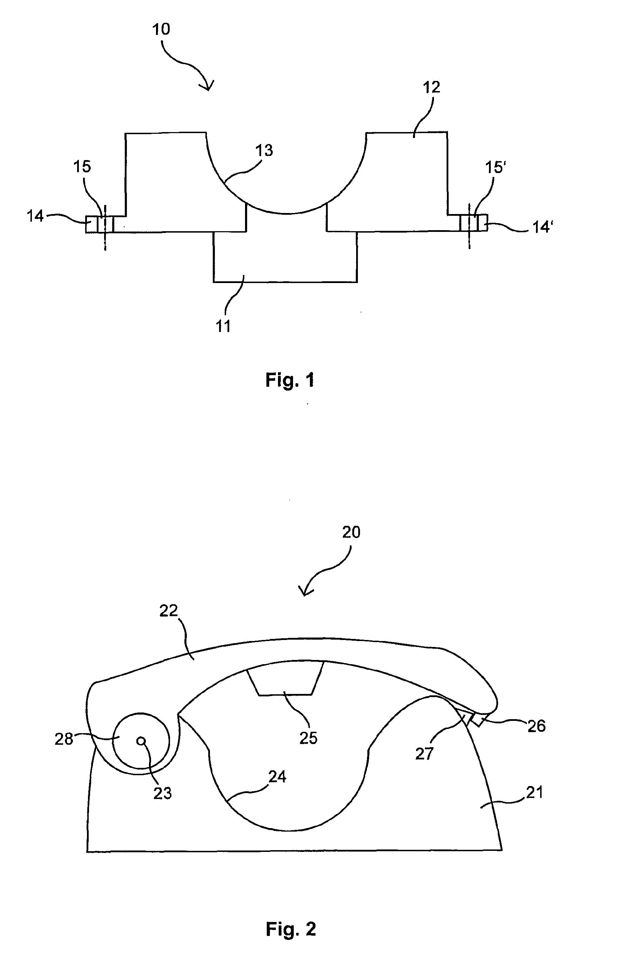

Fig. 1 zeigt eine schematische Seitenansicht eines Ausführungsbeispiels eines sensorspezifischen Bauteils 10. Das Bauteil wird im Wesentlichen durch einen Grundkörper 12 gebildet, in dem mittig eine Schlauchrinne 13 ausgeformt ist. Diese kann auch als Schlauchaufnahme bezeichnet werden, da sie zur Aufnahme und Ausrichtung des Schlauchs innerhalb der Sensoreinheit dient. Sie kann wie im dargestellten Ausführungsbeispiel einen in etwa halbkreisförmigen Querschnitt aufweisen. Der Querschnitt kann jedoch auch anders ausgeführt sein.

Unterhalb dieser Schlauchrinne 13 ist am bzw. im Grundkörper 12 die Sensorik 11 des sensorspezifischen Bauteils 10 angebracht, die auch als Messsystem bezeichnet werden kann. Diese Sensorik 11 unterscheidet sich von den Sensoriken anderer sensorspezifischer Bauteile und richtet sich nach dem Wirkprinzip der jeweiligen Sensoreinheit. Die Sensorik 11 muss jedoch nicht mittig direkt unterhalb der Schlauchrinne 13 angebracht sein, sondern sie kann beispielsweise auch von der Seite an einen in die Schlauchrinne 13 eingebrachten Schlauch ankoppeln. Dabei können sich die Formen der Schlauchrinnen der jeweiligen sensorspezifischen Bauteile unterscheiden. Ferner können sich die Formen der Grundkörper 12 der verschiedenen sensorspezifischen Bauteile 10 unterscheiden, solange sie so ausgebildet sind, dass ein einheitliches sensorneutrales Bauteil auf ihnen montierbar ist.

An den Seiten des Grundkörpers 12 sind Montagelaschen 14 und 14' ausgeformt, mit denen das sensorspezifische Bauteil 10 an dem Gehäuse eines medizinischen Geräts zur extrakorporalen Blutbehandlung angebracht werden kann (nicht dargestellt). Beispielsweise können hierzu Montagebohrungen 15 und 15' innerhalb der Montagelaschen 14, 14' vorgesehen sein. Bei Montage an einem Gehäuse befindet sich die Sensorik 11 vorzugsweise innerhalb des Gehäuses, so dass hierfür eine Aussparung im Gehäuse vorhanden sein muss. Der Grundkörper 12 hingegen ragt aus dem Gehäuse heraus.

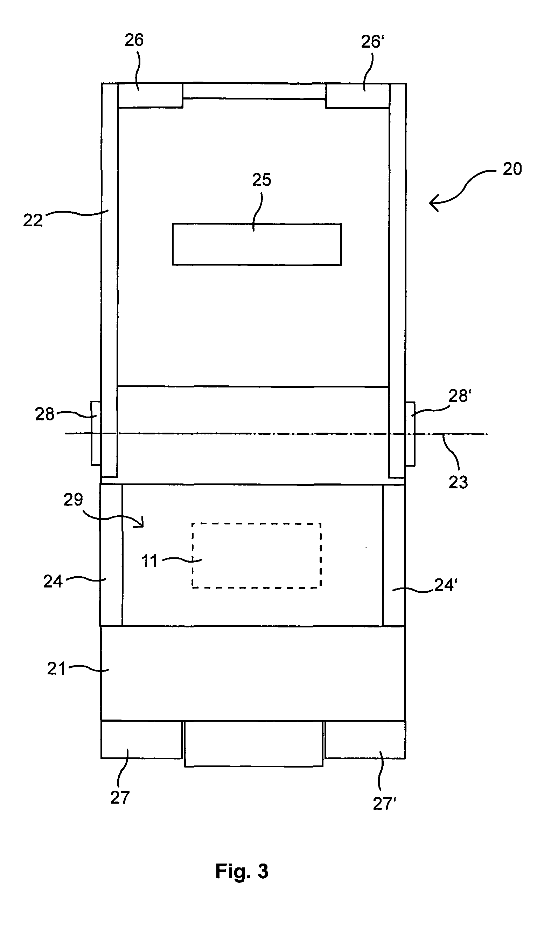

Fig. 2 zeigt eine schematische Seitenansicht eines Ausführungsbeispiels eines sensorneutralen Bauteils 20. Dieses Bauteil 20 besteht ebenfalls aus einem Grundkörper 21, in dem wenigstens eine rinnenförmige Schlauchhalterung ausgebildet ist. Allerdings handelt es sich hierbei nicht um eine durchgehende Rinne, sondern zwei schmale Rinnen 24 und 24', die durch eine Aussparung 29 voneinander beabstandet sind. Dies ist insbesondere der Aufsicht der Fig. 3 zu entnehmen. Fig. 3 zeigt dabei gestrichelt auch eine Sensorik 11 eines sensorspezifischen Bauteils 10 innerhalb der Aussparung 29, um darzustellen, wie die Sensorik 11 durch das sensorneutrale Bauteil 20 hindurch von einem Schlauch kontaktiert werden kann, der in das sensorneutrale Bauteil 20 eingelegt wird.

Das sensorneutrale Bauteil 20 weist ferner eine Abdeckung 22 auf, die schwenkbar an dem Grundkörper 21 des Bauteils 20 angebracht ist. Die schwenkbare Anbringung der Abdeckung 22 am Grundkörper 21 kann beispielsweise über zwei seitliche Bolzen 28 und 28' erfolgen. Die Abdeckung 22 ist so über eine Drehachse 23 gelagert, dass die Abdeckung 22 geöffnet werden kann, um den Abschnitt eines Schlauchs in die Schlauchhalterungen 24, 24' einbringen zu können. Bei eingebrachtem Schlauch kann die Abdeckung 22 durch Schwenken geschlossen und durch Rastelemente an dem Grundkörper 21 arretiert werden. Dazu sind am Grundkörper 21 beispielsweise zwei Rastnasen 27 und 27' angebracht, wie sie auch der Aufsicht der Fig. 3 zu entnehmen sind. An der Abdeckung 22 sind ebenfalls zwei Rastnasen 26 und 26' vorgesehen, die unter die Rastnasen 27, 27' des Grundkörpers 21 einrasten, wenn die Abdeckung 22 heruntergedrückt wird.

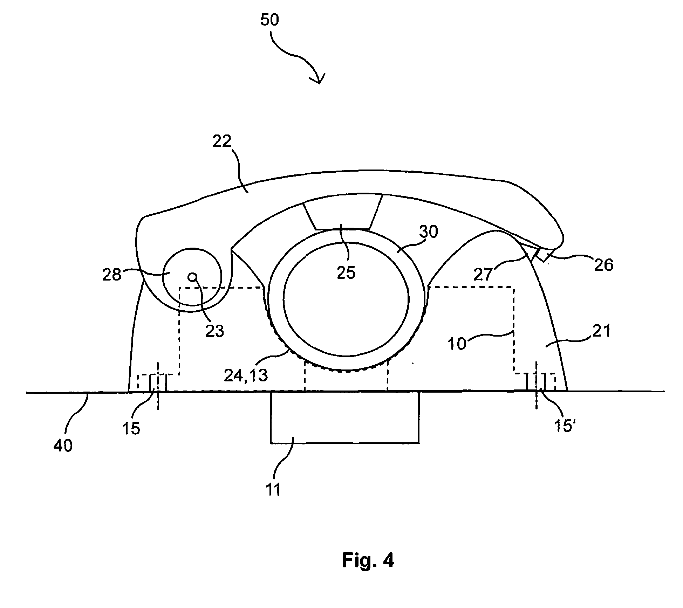

An der Innenseite der Abdeckung 22 kann ein Druckelement 25 ausgebildet sein, welches in der geschlossenen Stellung der Abdeckung 22 nach unten ragt. Hierdurch kann ein eingebrachter Schlauch in Richtung der Sensorik 11 eines sensorspezifischen Bauteils 10 gedrückt werden, denn das sensorneutrale Bauteil 20 kann vorzugsweise vollständig über das sensorspezifische Bauteil 10 gestülpt und ebenfalls am Gehäuse eines medizinischen Geräts zur extrakorporalen Blutbehandlung montiert werden. Dies ist in Fig. 4 gezeigt, wobei das unterhalb des sensorneutralen Bauteils 20 montierte sensorspezifische Bauteil 10 gestrichelt dargestellt ist.

Beide Bauteile 10 und 20 sind an einem Gehäuse 40 montiert und bilden zusammen eine Sensoreinheit 50. Für eine solche Montage können an dem sensorneutralen Bauteil 20 ebenfalls Montagebohrungen oder andere Befestigungsmittel vorgesehen sein. Dabei kann das sensorneutrale Bauteil an dem Gehäuse befestigt werden, oder das sensorneutrale Bauteil wird an dem sensorspezifischen Bauteil fixiert, welches dann wiederum am Gehäuse befestigt ist.

Erfindungsgemäß sind wenigstens zwei dieser Sensoreinheiten 50 an einem medizinischen Gerät zur extrakorporalen Blutbehandlung angebracht, wobei die außen liegenden sensorneutralen Bauteile 20 immer identisch sind, während sich die darunter liegenden sensorspezifischen Bauteile 10 je nach dem Wirkprinzip der zugehörigen Sensorik 11 unterscheiden. Für den Bediener stellt sich jedoch jede Sensoreinheit 50 gleich dar, so dass er für das Einlegen und Fixieren eines Schlauchs 30 innerhalb einer Sensoreinheit 50 keine Kenntnis über die jeweilige Sensorik 11 haben muss. Unabhängig von der Sensorik 11 kann der Bediener den Schlauch 30 in jede Sensoreinheit 50 gleich einlegen und fixieren, da dies von dem außen liegenden sensorneutralen Bauteil 20 vorgegeben wird, und die Ankopplung an das darunter liegende sensorspezifische Bauteil 10 wird durch ein entsprechendes Ineinandergreifen der beiden Bauteile realisiert.

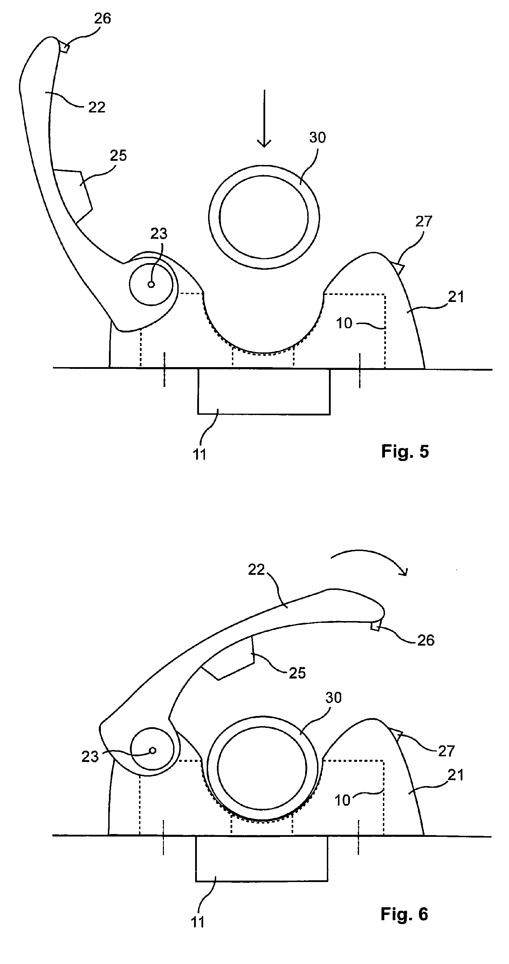

Für ein Einlegen des Schlauchs öffnet der Bediener die Abdeckung 22, indem er sie um die Drehachse 23 schwenkt und legt den Schlauch 30 in die Sensoreinheit 50 ein, wie es in Fig. 5 durch einen Pfeil nach unten dargestellt ist. Dabei liegen die Schlauchhalterungen 24 und 24' des sensorneutralen Bauteils 20 an den beiden Seiten der Schlauchrinne 13 des sensorspezifischen Bauteils 10 an, so dass sich dem Bediener von oben eine durchgehende Schlauchrinne darbietet, in welche er den Schlauch 30 einlegen kann. Anschließend wird die Abdeckung 22 in die geschlossene Stellung geschwenkt, wie es in Fig. 6 durch einen gebogenen Pfeil gezeigt ist. Sobald das Druckelement 25 Kontakt zu dem eingelegten Schlauch 30 hat, drückt es diesen gegen die Sensorik 11 des sensorspezifischen Bauteils 10, so dass ein sicherer Kontakt zwischen dem Schlauch 30 und der Sensorik 11 hergestellt ist. Ferner rastet die Abdeckung 22 über die Rastnasen 26, 26', 27 und 27' am Grundkörper 21 ein. Um den Schlauch 30 nach einer Therapie wieder aus der Sensoreinheit 50 zu entnehmen, ist die Verrastung so ausgeführt, dass die Abdeckung 22 wieder manuell aus dieser gelöst werden kann.

### Bezugszeichenliste

- 10: Sensorspezifisches Bauteil
- 11: Sensorik
- 12: Erster Grundkörper
- 13: Schlauchrinne, Schlauchaufnahme
- 14,14': Montagelasche
- 15,15': Montagebohrung
- 20: Sensorneutrales Bauteil
- 21: Zweiter Grundkörper
- 22: Abdeckung
- 23: Drehachse
- 24,24': Schlauchhalterung
- 25: Druckelement
- 26,26',27,27': Rastelement, Rastnase
- 28: Bolzen
- 29: Aussparung
- 30: Schlauch
- 40: Gehäuse
- 50: Sensoreinheit

## Patentansprüche

1. Medizinisches Gerät zur extrakorporalen Blutbehandlung, umfassend einen extrakorporalen Blutkreislauf mit wenigstens einem Schlauch (30) und wenigstens zwei auf dem Schlauch messende Sensoreinheiten (50) mit unterschiedlichen Wirkprinzipien, an denen Abschnitte des Schlauchs (30) für eine Messung ankoppelbar sind,
wobei
jede Sensoreinheit (50) aus einem sensorspezifischen Bauteil (10) und einem sensorneutralen Bauteil (20) besteht,
die sensorneutralen Bauteile (20) der Sensoreinheiten (50) identisch ausgebildet sind, während sich die sensorspezifischen Bauteile (10) abhängig vom Wirkprinzip unterscheiden und jeweils eine spezifische Sensorik (11) umfassen,
für jede Sensoreinheit (50) das jeweilige sensorneutrale Bauteil (20) auf dem entsprechenden sensorspezifischen Bauteil (10) montiert ist, und
ein Abschnitt des Schlauchs (30) bei Einbringung und Fixierung in einem der sensorneutralen Bauteile (20) so an die entsprechende Sensorik (11) des darunter liegenden sensorspezifischen Bauteils (10) ankoppelbar ist, dass mit der entsprechenden Sensorik (11) eine Messung am Schlauch (30) durchführbar ist.

2. Medizinisches Gerät nach Anspruch 1,
**dadurch gekennzeichnet, dass** die sensorneutralen und sensorspezifischen Bauteile (10;20) jeweils unterschiedlich ausgeformte Grundkörper (12;21) aufweisen, und die Grundkörper (21) der sensorneutralen Bauteile (20) jeweils die darunter liegenden Grundkörper (12) der sensorspezifischen Bauteile (10) umschließen.

3. Medizinisches Gerät nach Anspruch 2,
**dadurch gekennzeichnet, dass** die Grundkörper (21) der sensorneutralen Bauteile (20) eine Aussparung (29) aufweisen, und ein Abschnitt des Schlauchs (30) so in die sensorneutralen Bauteile (20) einbringbar ist, dass der Abschnitt des Schlauchs (30) durch die Aussparung (29) hindurch an die Sensorik (11) eines sensorspezifischen Bauteils (10) ankoppelbar ist.

4. Medizinisches Gerät nach Anspruch 3,
**dadurch gekennzeichnet, dass** jedes sensorspezifische Bauteil (10) eine Schlauchrinne (13) aufweist, in welche der Abschnitt eines Schlauchs (30) einlegbar ist.

5. Medizinisches Gerät nach Anspruch 4,
**dadurch gekennzeichnet, dass** die Schlauchrinnen (13) von wenigstens zwei sensorspezifischen Bauteilen (10) unterschiedlich ausgebildet sind.

6. Medizinisches Gerät nach einem oder beiden der Ansprüche 4 und 5,
**dadurch gekennzeichnet, dass** jedes sensorneutrale Bauteil (20) zwei rinnenförmige Schlauchhalterungen (24;24') mit der dazwischen liegenden Aussparung (29) aufweist, und die Schlauchhalterungen (24;24') so an den beiden Enden der Schlauchrinne (13) des sensorspezifischen Bauteils (10) liegen, dass sich eine durchgehende Schlauchrinne ergibt.

7. Medizinisches Gerät nach einem oder mehreren der Ansprüche 1 bis 6,
**dadurch gekennzeichnet, dass** jedes sensorneutrale Bauteil (20) eine Abdeckung (22) aufweist, mit welcher der Abschnitt eines Schlauchs (30) in einer geschlossenen und arretierten Stellung gegen die Sensorik (11) des zugehörigen sensorspezifischen Bauteils (10) drückbar ist.

8. Medizinisches Gerät nach Anspruch 7,
**dadurch gekennzeichnet, dass** die Abdeckung (22) wenigstens ein an der Abdeckung (22) hervorstehendes Druckelement (25) aufweist, über welches Druck auf den Abschnitt eines Schlauchs (30) aufbringbar ist.

9. Medizinisches Gerät nach einem oder beiden der Ansprüche 7 und 8,
**dadurch gekennzeichnet, dass** die Abdeckung (22) schwenkbar an dem jeweiligen Grundkörper (21) des sensorneutralen Bauteils (20) angebracht und in einer geschlossenen Stellung an dem Grundkörper (21) arretierbar ist.

10. Medizinisches Gerät nach Anspruch 9,
**dadurch gekennzeichnet, dass** für die Arretierung an der Abdeckung (22) und dem Grundkörper (21) jeweils wenigstens ein Rastelement (26;26';27;27') vorgesehen ist.

## Claims

1. A medical device for extracorporeal blood treatment comprising an extracorporeal blood circulation having at least one tube (30) and at least two sensor units (50) measuring on the tube with different operating principles, wherein portions of the tube (30) are adapted to be coupled thereto for measurement,
wherein
each sensor unit (50) consists of a sensor-specific component (10) and a sensor-neutral component (20),
the sensor-neutral components (20) of the sensor units (50) being formed to be identical, whereas the sensor-specific components (10) are different dependent on the respective operating principle and comprise a respective specific sensor system (11),
for each sensor unit (50) a sensor-neutral component (20) is mounted on a sensor-specific component (10), and
upon being introduced and fixed in a sensor-neutral component (20) a portion of the tube (30) is adapted to be coupled to the respective sensor system (11) of the sensor-specific component (10) located beneath so that the respective sensor system (11) can carry out a measurement at the tube (30).

2. The medical device according to claim 1,
**characterized in that** the sensor-neutral and sensor-specific components (10; 20) each have differently formed base members (12; 21) and the base members (21) of the sensor-neutral components (20) enclose the base members (12) of the sensor-specific components (10) located underneath.

3. The medical device according to claim 2,
**characterized in that** the base members (21) of the sensor-neutral components (20) include a recess (29) and a portion of the tube (30) is adapted to be introduced into the sensor-neutral components (20) so that the portion of the tube (30) is adapted to be coupled to the sensor system (11) of a sensor-specific component (10) through the recess (29).

4. The medical device according to claim 3,
**characterized in that** each sensor-specific component (10) includes a tube channel (13) into which the portion of a tube (30) can be inserted.

5. The medical device according to claim 4,
**characterized in that** the tube channels (13) of at least two sensor-specific components (10) are formed differently.

6. The medical device according to either or both of the claims 4 and 5,
**characterized in that** each sensor-neutral component (20) has two channel-type tube holders (24; 24') with the recess (29) located there between and that the tube holders (24; 24') are located at the two ends of the tube channel (13) of the sensor-specific component (10) so that a continuous tube channel is resulting.

7. The medical device according to one or more of the claims 1 to 6,
**characterized in that** each sensor-neutral component (20) has a cover (22) by which in a closed and locked position the portion of a tube (30) is adapted to be pressed against the sensor system (11) of the associated sensor-specific component (10).

8. The medical device according to claim 7,
**characterized in that** the cover (22) includes at least one pressure element (25) protruding from the cover (22) by which pressure is applicable to the portion of a tube (30).

9. The medical device according to either or both of the claims 7 and 8,
**characterized in that** the cover (22) is adapted to be pivoted to the respective base member (21) of the sensor-neutral component (20) and is adapted to be locked in a closed position at the base member (21).

10. The medical device according to claim 9,
**characterized in that** at least one detent element (26, 26'; 27; 27') is provided for locking at the cover (22) and at the base member (21).

## Revendications

1. Appareil médical pour le traitement extracorporel du sang, comprenant un circuit de sang extracorporel avec au moins un tuyau (30) et au moins deux unités de capteurs de mesure (50) prévus sur le tuyau, avec différents principes d'action, qui peuvent être couplés sur des portions du tuyau (30) pour une mesure,
chaque unité de capteur (50) étant constituée d'un composant spécifique au capteur (10) et d'un composant neutre de capteur (20),
les composants neutres de capteur (20) des unités de capteurs (50) étant conçus de manière identique tandis que les composants spécifiques au capteur (10) sont différents du point de vue du principe de fonctionnement et comprennent chacun les technologies de capteur spécifique (11),
pour chaque unité de capteur (50), le composant neutre de capteur (20) étant monté sur le composant spécifique au capteur (10) correspondant et
une portion du tuyau (30) pouvant être couplée, lors de l'insertion et de la fixation dans un des composants neutres de capteur (20), aux technologies de capteur spécifique correspondantes (11) du composant spécifique au capteur (10) situé en dessous de façon à ce que, avec les technologies de capteur spécifique (11) correspondantes, une mesure est réalisable sur le tuyau (30).

2. Appareil médical selon la revendication 1,
**caractérisé en ce que** les composants neutres et spécifiques du capteur (10 ; 20) comprennent chacun des corps de base (12 ; 21) de formes différentes et les corps de base (21) des composants neutres de capteurs (20) entourent chacun les corps de base (12), situés en dessous, des composants spécifiques au capteur (10).

3. Appareil médical selon la revendication 2,
**caractérisé en ce que** les corps de base (21) des composants neutres du capteur (20) comprennent un évidement (29) et une portion du tuyau (30) peut être insérée dans les composants neutres du capteur (20) de façon à ce que la portion du tuyau (30) puisse être couplée par l'intermédiaire de l'évidement (29) aux technologies de capteur spécifique (11) d'un composant spécifique au capteur (10).

4. Appareil médical selon la revendication 3,
**caractérisé en ce que** chaque composant spécifique au capteur (10) comprend une rigole de tuyau (13) dans laquelle la portion d'un tuyau (30) peut être insérée.

5. Appareil médical selon la revendication 4,
**caractérisé en ce que** les rigoles de tuyaux (13) sont conçues différemment à partir d'au moins deux composants spécifique au capteur (10).

6. Appareil médical selon l'une ou les deux revendications 4 et 5,
**caractérisé en ce que** chaque composant neutre du capteur (20) comprend deux supports de tuyaux (24 ; 24') en forme de rigole avec l'évidement (29) situé entre ceux-ci et les supports de tuyaux (24 ; 24') sont disposés au niveau des deux extrémités de la rigole de tuyau (13) du composant spécifique au capteur (10), de façon à ce qu'il en résulte une rigole de tuyau continue.

7. Appareil médical selon l'une ou plusieurs des revendications 1 à 6,
**caractérisé en ce que** chaque composant neutre du capteur (20) comprend un couvercle (22), avec lequel la portion d'un tuyau (30) peut être comprimée dans une position fermée et bloquée contre les technologies de capteur spécifique (11) du composant spécifique au capteur (10) correspondant.

8. Appareil médical selon la revendication 7,
**caractérisé en ce que** le couvercle (22) comprend au moins un élément de compression protubérant (25) dépassant du couvercle (22), qui permet d'exercer une pression sur la portion d'un tuyau (30).

9. Appareil médical selon l'une ou les deux revendications 7 et 8,
**caractérisé en ce que** le couvercle (22) est monté de manière pivotante sur le corps de base (21) correspondant du composant neutre du capteur (20) et peut être bloqué dans une position fermée sur le corps de base (21).

10. Appareil médical selon la revendication 9,
**caractérisé en ce que**, pour le blocage, au moins un élément d'encliquetage (26 ; 26' ; 27 ; 27') est prévu sur le couvercle (22) et le corps de base (21).
